# EUROPEAN PATENT APPLICATION

(11) **EP 2 980 807 A1**
(43) Date of publication of application: **03.02.2016**
(21) Application number: 14382301.1
(22) Date of filing: 31.07.2014
(51) Int. Cl.: H01F 1/00, B82Y 30/00, A61K 49/00, A61K 51/12, A61K 49/18, B82B 3/00

(54) **Single core radionuclide-metal oxide nanoparticles: a new biocompatible nanosystem for dual hot spot imaging**

(71) Applicant: CNIC Fundación Centro Nacional de Investigaciones Cardiovasculares Carlos III, 28029 Madrid (ES); Universidad Complutense De Madrid, 28040 Madrid (ES)
(72) Inventor: Herranz Rabanal, Fernando, 28029 Madrid (ES); Ruiz-Cabello Osuna, Jesús, 28029 Madrid (ES); Pellico Sáez, Juan, 28040 Madrid (ES); Bhavesh, Riju, 28029 Madrid (ES)
(74) Representative: Hoffmann Eitle

(57) **Abstract**

The present invention shows extremely small iron oxide nanoparticles, 2 nm core size, ⁶⁸Ga core-doped through a microwave driven synthesis that provides an expedited protocol with radiolabelling efficiency yield of around 90%, optimal for short half-lived isotopes. *In vivo* hot spot MRI (T₁ weighted contrast) and PET imaging are demonstrated with unprecedented quality for iron oxide-based dual modality probes. The results incorporated herein show, for the first time, a new concept for dual-modality nanoparticle production and effective probe production with single core integration of both sources of contrast that will surely boost a large number of cardiovascular applications.

## Description

### Technical field of the invention

The present invention refers to the medical field, in particular to the field of medical diagnosis, more in particular to the use of single core radionuclide-Fe3O4 nanoparticles for dual hot spot imaging.

### Background of the invention

Iron Oxide nanoparticles (IONPs) main features for biomedical applications, superparamagnetism and biocompatibility, are well-known and they have made this nanomaterial one of the most used in biomedicine. They are used as MRI (Magnetic Resonance Imaging) T₂-weighted MRI, producing a darkening of the tissue where they accumulate. This is one of the major drawbacks of these nanoparticles. The effect of negative contrast makes it many times harder to differentiate the signal from image artifacts and the natural low signal of some organs like the lungs. This fact has encouraged many research groups within this field to seek for particles able to produce positive, T₁-weighted, MRI contrast synthesized by hydrothermal synthesis. These groups have reported the synthesis of small IONPs by traditional, lengthy protocols, from 3 to 24 hours of reaction time, making them unsuitable for radionuclide labeling, especially with short-lived isotopes.

Therefore, there is still a need to obtain dual Consistent IONPs Nanoparticles capable of providing in vivo signals for PET and positive contrast MRI. This type of nanoparticles will certainly open a complete new range of applications in different fields, in particular in the medical field.

### Brief description of the invention

A first aspect of the invention refers to a preparation method of metal oxide nanoparticles, preferably metal oxides from the following list of metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C, preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃,
   b. a biodegradable hydrophilic polymer, wherein preferably the hydrophilic polymer is dextran (preferably of 6kDal),
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the first aspect of the invention refers to a preparation method of metal oxide nanoparticles, preferably metal oxides from the following list of metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol; and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the first aspect of the invention refers to a preparation method of metal oxide nanoparticles, preferably metal oxides from the following list of metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol; and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature of about 100°C, with microwave irradiation at about 240W for about 10 min; and
c. Purifying the nanoparticles obtained in step b).

In a preferred embodiment of the first aspect of the invention or of any of its preferred embodiments, the purification step is carried out by gel filtration chromatography.

A second aspect of the invention refers to the nanoparticles obtained or obtainable by the preparation method of the first aspect of the invention or of any of its preferred embodiments.

A third aspect of the invention refers to a metal oxide nanoparticle, preferably an iron oxide nanoparticle, which comprises:
a. A surface layer of a biodegradable hydrophilic polymer; and
b. A core comprising a metal oxide, preferably a metal oxide selected from the following list of metals Fe, Al, B, Co, Zn, Ni, Zr, Ga, Y and Yb, more preferably the metal oxide is an iron oxide (such as FeO, Fe₃O₄, Fe₄O₅, Fe₂O₃ or FeO₃, still more preferably the iron oxide is Fe₃O₄), and radionuclide suitable for medical imaging;
wherein the nanoparticle is characterized by having the following values:
a. r1 is from 3-10 mM⁻¹s⁻¹as measured by relaxometry,
b. r2 is from 11-40 mM⁻¹s⁻¹as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 20 +/-10 as measured by a Superconducting Quantum Interference Device (SQUID).

A preferred embodiment of the third aspect of the invention refers to a metal oxide nanoparticle, preferably an iron oxide nanoparticle, which comprises:
a. A surface layer of a biodegradable hydrophilic polymer, preferably dextran (6kDal); and
b. A core comprising a metal oxide, preferably a metal oxide selected from the following list of metals Fe, Al, B, Co, Zn, Ni, Zr, Ga, Y and Yb, more preferably the metal oxide is an iron oxide (such as FeO, Fe₃O₄, Fe₄O₅, Fe₂O₃ or FeO₃, still more preferably the iron oxide is Fe₃O₄), and radionuclide selected from the list consisting of C-11, N-13, O-15, F-18, Cu-64, Ga-68, Ga-67, Tc-99, Ho-166, In-111, Lu-177, Yb-169, Bi-213, Er-169, Y-90 and T1-201 and Zr-89,
wherein the nanoparticle is characterized by having the following values:
a. r1 is from 3-10 mM⁻¹s⁻¹as measured by relaxometry
b. r2 is from 11-40 mM⁻¹s⁻¹as measured by relaxometry
c. a core size of from 1.5 - 3 nm, according to Transmission Electron Microscopy measurement,
d. a hydrodynamic size of from 20± 5 nm by DLS, and
e. a superparamagnetic behavior with a value of magnetic saturation (Ms) of from 22 +/-10 as measured by a Superconducting Quantum Interference Device (SQUID).

Another preferred embodiment of the third aspect of the invention refers to a metal oxide nanoparticle, preferably an iron oxide nanoparticle, which comprises:
a. A surface layer of a biodegradable hydrophilic polymer, preferably dextran (6 kDal); and
b. A core comprising a metal oxide, preferably a metal oxide selected from the following list of metals Fe, Al, B, Co, Zn, Ni, Zr, Ga, Y and Yb, more preferably the metal oxide is an iron oxide (such as FeO, Fe₃O₄, Fe₄O₅, Fe₂O₃ or FeO₃, still more preferably the iron oxide is Fe₃O₄), and radionuclide selected from the list consisting of C-11, N-13, O-15, F-18, Cu-64, Ga-68, Ga-67, Tc-99, Ho-166, In-111, Lu-177, Yb-169, Bi-213, Er-169, Y-90 and T1-201 and Zr-89, preferably Ga-68 or Zr-89.
wherein the nanoparticle is characterized by having the following values:
a. r1 is from 3-10 mM⁻¹s⁻¹as measured by relaxometry
b. r2 is from 11-40 mM⁻¹s⁻¹as measured by relaxometry
c. a core size of from 1.5 - 3 nm, according to Transmission Electron Microscopy measurement,
d. a hydrodynamic size of from 20± 5 nm by DLS, and
e. a superparamagnetic behavior with a value of magnetic saturation (Ms) of from 22 +/-2 as measured by a Superconducting Quantum Interference Device (SQUID).

Another preferred embodiment of the third aspect of the invention refers to a metal oxide, preferably an iron oxide nanoparticle, which comprises:
a. A surface layer of dextran; and
b. A core comprising an iron oxide (such as FeO, Fe₃O₄, Fe₄O₅, Fe₂O₃ or FeO₃, still more preferably the iron oxide is Fe₃O₄), and a radionuclide selected from the list consisting of Zr-89 and Ga-68, preferably Ga-68.
wherein the nanoparticle is characterized by having the following values:
a. r1 is from 3-10 mM⁻¹s⁻¹as measured by relaxometry
b. r2 is from 11-40 mM⁻¹s⁻¹as measured by relaxometry
c. a core size of from 1.5 - 3 nm, according to Transmission Electron Microscopy measurement,
d. a hydrodynamic size of from 20± 5 nm by DLS, and
e. a superparamagnetic behavior with a value of magnetic saturation (Ms) of from 22 +/-2 as measured by a Superconducting Quantum Interference Device (SQUID).

A fourth aspect of the invention refers to a preparation method of metal oxide nanoparticles, preferably metal oxides from the following metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C, preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃,
   b. a biodegradable hydrophilic polymer, wherein preferably the hydrophilic polymer is dextran (preferably of 6kDal),
   c. a radionuclide suitable for medical imaging preferably selected from the group consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89, more preferably Ga-68 or Zr-89,
   d. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the fourth aspect of the invention refers to a preparation method of metal oxide nanoparticles, preferably metal oxides from the following metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol;
   c. a radionuclide suitable for medical imaging preferably selected from the group consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89, more preferably Ga-68 or Zr-89, in an amount between 0.5 mCi y 60 mCi, more preferably between 0.5 and 40 mCi; and
   d. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the fourth aspect of the invention refers to a preparation method of metal oxide nanoparticles, preferably metal oxides from the following metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol;
   c. a radionuclide suitable for medical imaging preferably selected from the group consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89, more preferably Ga-68 or Zr-89, in an amount between 0.5 mCi y 60 mCi, more preferably between 0.5 and 40 mCi; and
   d. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature of about 100°C, with microwave irradiation at about 240W for about 10 min; and
c. Purifying the nanoparticles obtained in step b).

A fifth aspect of the invention refers to an iron oxide nanoparticle obtained or obtainable by the preparation method of the fourth aspect of the invention.

A sixth aspect of the invention refers to the nanoparticles of the second, third or fifth aspect of the invention for use as a medicament, or for use in therapy or medical diagnosis.

A seventh aspect of the invention refers to the nanoparticles of the second, third or fifth aspect of the invention for use in a method of diagnosis and/or therapy capable of providing in vivo signals for PET (Positron Emission Tomography) and/or positive contrast MRI (Magnetic Resonance Imaging). An eight aspect of the invention refers to the use of the nanoparticles of the second, third or fifth aspect of the invention in a method capable of providing in vivo signals for PET (Positron Emission Tomography) and/or positive contrast MRI (Magnetic Resonance Imaging).

A ninth aspect of the invention refers to the nanoparticles of the second, third or fifth aspect of the invention for use in a method of diagnosis and/or therapy via systemic administration, preferably intravenous administration, capable of providing in vivo signals for PET (Positron Emission Tomography) and/or positive contrast MRI (Magnetic Resonance Imaging).

It is noted that in the context of the present invention the longitudinal (or spin-lattice) relaxation time *T*₁ is the decay constant for the recovery of the z component of the nuclear spin magnetization, *M_{z},* towards its thermal equilibrium value. The transverse (or spin-spin) relaxation time *T*₂ is the decay constant for the component of the magnetization perpendicular to the external magnetic field.

### Brief description of the figures

**Figure 1****.** Physicochemical characterization of dCNIC-Dextran. A) TEM images at two magnifications. B) Hydrodynamic size measured by DLS. C) FTIR spectrum of dCNIC-Dextran. D) Field dependent magnetization.
**Figure 2****.** A) Plots of T₁ and T₂ relaxation rates against iron concentration. B) T₁-weighted MRI phantoms of dCNIC-Dextran nanoparticles.
**Figure 3****.** a) Procedure for the synthesis of ⁶⁸Ga-dCNIC-Dextran by microwave irradiation. b) TEM images of ⁶⁸Ga-dCNIC-Dextran. c) Comparison of average hydrodynamic size (N=5) for dCNIC-Dextran and ⁶⁸Ga-dCNIC-Dextran. d) Typical FTIR spectra for dCNIC-Dextran and ⁶⁸Ga-dCNIC-Dextran.
**Figure 4****.** a) Chromatogram for ⁶⁸Ga-dCNIC-Dextran in the UV channel at 380 nm (black line) and the radioactivity channel (red line), compared with the chromatogram for free ⁶⁸GaCl₃ (green line). b) Plot of T₁ and T₂ relaxation rates against iron concentration. c) Image phantoms obtained at different iron concentrations by MRI (top row) and PET (bottom row).
**Figure 5****.** Magnetic resonance angiography (MRA) of a rabbit before i.v. injection of dCNIC-Dextran (Basal) and at 6 minutes and 1 hour post injection.
**Figure 6****.** XRD spectra of dCNIC-Dextran (bottom line), ⁶⁸Ga-dCNIC-Dextran (2 mCi, middle line) and ⁶⁸Ga-dCNIC (1 mCi, top line)
**Figure 7**. Hydrodynamic size of five preparations of dCNIC-Dextran in saline.
**Figure 8****.** Thermogravimetric curve for dCNIC-Dextran sample (Left) and 68Ga-dCNIC (right).
**Figure 9****.** Size exclusion chromatography elution profile. The first large eluted fraction corresponds to ⁶⁸Ga-dCNIC-Dextran particles, and is followed by a small peak due to elution of unreacted free ⁶⁸Ga.
**Figure 10****.** Magnetization curves for ⁶⁸Ga-dCNIC-Dextran and dCNIC-Dextran, showing no major change upon radiolabeling.
**Figure 11****.** MR angiography of a mouse at increasing times after intravenous injection of dCNIC-Dextran.
**Figure 12****.** PET/CT image of ⁶⁸Ga-dCNIC-Dextran injected into a mouse, followed, after decay, by MR angiography of the same mouse. PET detects vena cava and heart chambers, while MRA detects aorta, jugulars, carotids and several other small-diameter vessels.
**Figure 13****.** Process flow for integrated ⁶⁸Ga elution and microwave synthesis of dual hot-spot nanoparticles.
**Figure 14****.** MRI and PET phantoms for ⁸⁹Zr-dCNIC-Dextran, showing correlation of PET signal strength with positive-contrast MRI.
**Figure 15****.** In vivo biodistribution of the ⁸⁹Zr-dCNIC-Dextran signal after injection.

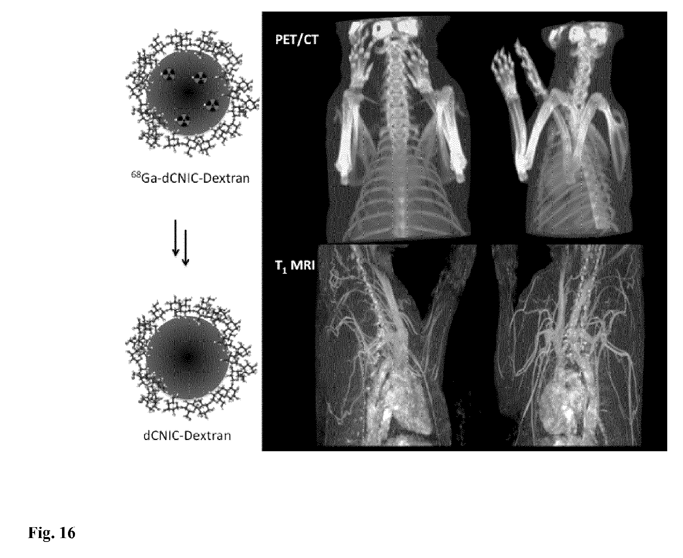

**Figure 16****.** PET/CT imaging in a rabbit (upper panels) 30 minutes after injection of ⁶⁸Ga-dCNIC-Dextran, showing strong activity in the heart chambers and the aorta. The lower panel show high-resolution MRI analysis in the same rabbit after the decay of ⁶⁸Ga. PET image is presented as a maximum intensity projection (MIP).

### Detailed description of the invention

The authors of the present invention confronted the problem of obtaining dual consistent IONPs especially capable of providing in vivo signals for PET and positive contrast MRI. For this purpose they hypothesized that the use of microwave synthesis (MWS) would allow not only to label the core of IONPs in very short times, even for applications with short-lived radioisotopes such as ⁶⁸Ga, but also that the use of low molecular weight dextran and the right conditions in the microwave synthesis could render very small nanoparticles able to provide T₁-weighted images in MRI, overcoming the major limitation of these nanoparticles.

In order to determine whether this hypothesis was correct, the authors of the present invention synthesized Dextran nanoparticles as shown in example 2.

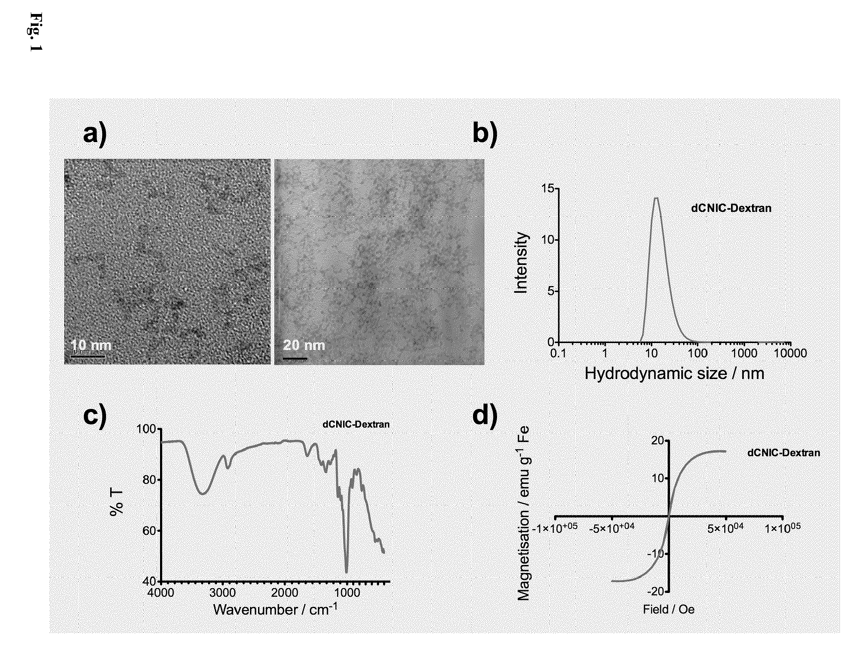

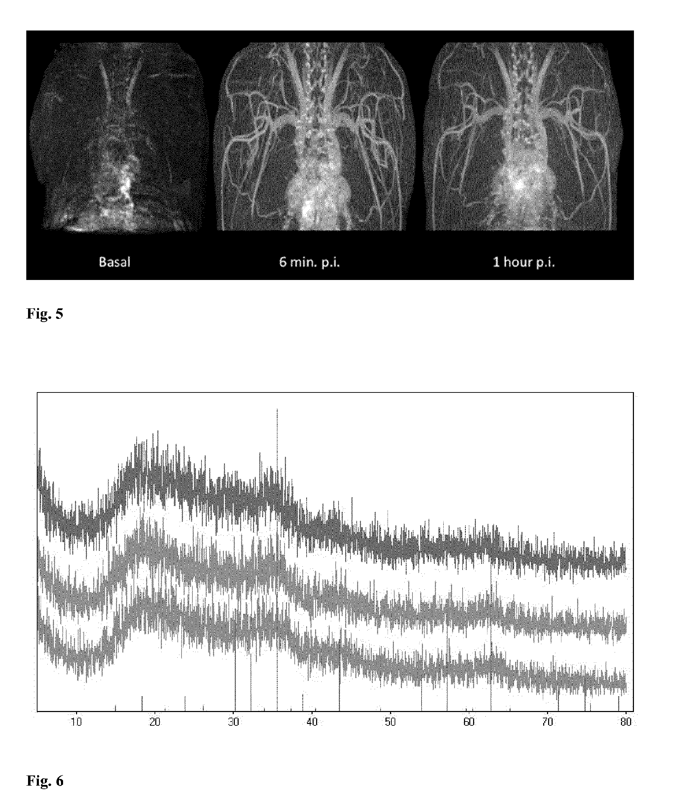

This approach rendered very small nanoparticles with a core size of about 2.1 ± 0.2 nm, according to TEM, and 2.6 nm of crystal size, according to XRD measurements (Figure 1a and Figure 6 respectively). Hydrodynamic size was also checked by DLS showing a value of 18.2 ± 2.5 nm (Figure 1b, Table 1).

Once the nanoparticles were synthesized the authors of the present invention measured the magnetic properties of the synthesized particles using a Superconducting Quantum Interference Device (SQUID), the results are shown in Figure 1d, confirming the superparamagnetic behavior with a value of Ms of 17.2 emu/g Fe. These data confirm that due to the rapid heating and therefore nucleation in the MWS, extremely small Fe₃O₄ particles can be produced.

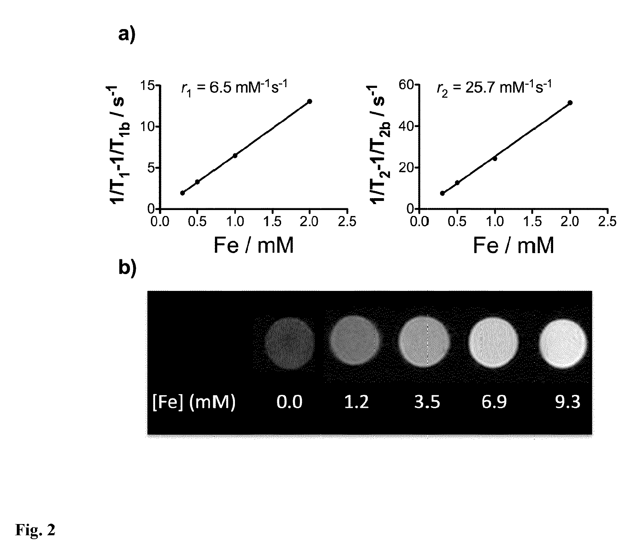

To examine the possibility of using the new synthesized dCNIC-Dextran particles as T₁ contrast agents in MRI, the relaxivity values, at 1.5 T and 37 °C, were measured. In order to use Fe₃O₄ nanoparticles as T₁ contrast agent, they must possess a high *r*₁ value and as low as possible *r*₂/*r*₁ ratio. The experimental values and linear fitting are shown in figure 2a and Table 1 with a very high value for *r*₁ of 6.0 mM⁻¹s⁻¹ and a low *r*₂ value of 22.7 mM⁻¹s⁻¹.

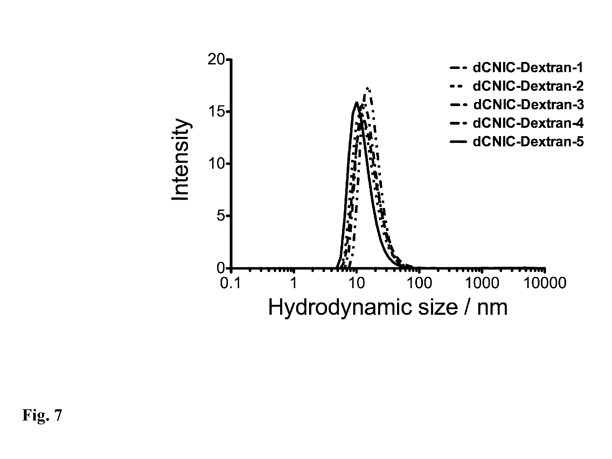

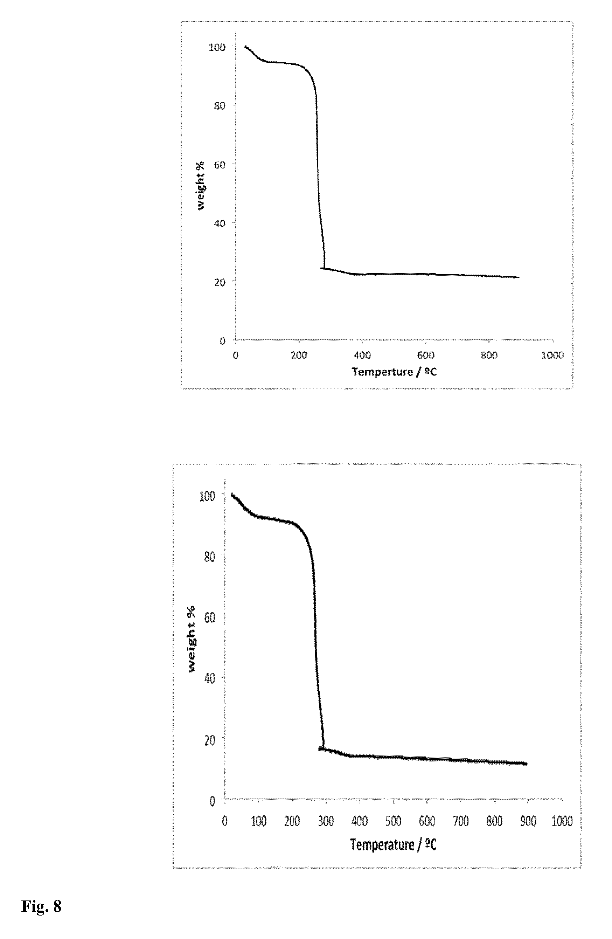

To confirm the possibility of generating positive contrast with this new approach, several phantoms were prepared at different Fe concentrations and MRI recorded. As can be seen in figure 2b, with a T₁-weighted sequence, a strong positive contrast is obtained as the Fe concentration increase. As far as we know these are the first iron oxide nanoparticles synthesized by microwave radiation showing capabilities as T₁ contrast agents. Furthermore one of the strongest points of this technology is the reproducibility and rapidity to increase the production rate. As can be seen in figure 7 the DLS measurement for 5 different syntheses showed nanoparticles with the same hydrodynamic size and narrow size distribution (indicate time) and also with a very high amount of Dextran on the surface, ensuring colloidal stability (TGA data in Figure 8). The same is true for the main physicochemical properties of the nanoparticles. Table 1 gathered some of the parameters for five repetitions of the synthesis confirming the reproducibility in all aspects, particularly in the relaxivity values and the *r*₂ to *r*₁ ratio, compulsory for their *in vivo* use.

Thus, a first aspect of the invention refers to a preparation method of metal oxide nanoparticles, preferably metal oxides from the following list of metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C, preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃,
   b. a biodegradable hydrophilic polymer, wherein preferably the hydrophilic polymer is dextran (preferably of 6kDal), and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the first aspect of the invention refers to a preparation method of metal oxide nanoparticles, preferably metal oxides from the following list of metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol; and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the first aspect of the invention refers to a preparation method of metal oxide nanoparticles, preferably metal oxides from the following list of metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol; and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature of about 100°C, with microwave irradiation at about 240W for about 10 min; and
c. Purifying the nanoparticles obtained in step b).

In a preferred embodiment of the first aspect of the invention, the purification step is carried out by gel filtration chromatography.

In the context of the present invention the term "about" is understood to indicate a value +/- 1% of the valued referred to therein.

In the context of the present invention the relaxivity (r1 or r2) of a contrast agent in water is the change in 1/T1 or 1/T2 of water per concentration of contrast agent.

In the context of the present invention a strong base is a basic chemical compound that can remove a proton (H+) from (or deprotonate) a molecule of a very weak acid in an acid-base reaction.

A second aspect of the invention refers to the nanoparticles obtained or obtainable by the preparation method of the first aspect of the invention or of any of its preferred embodiments.

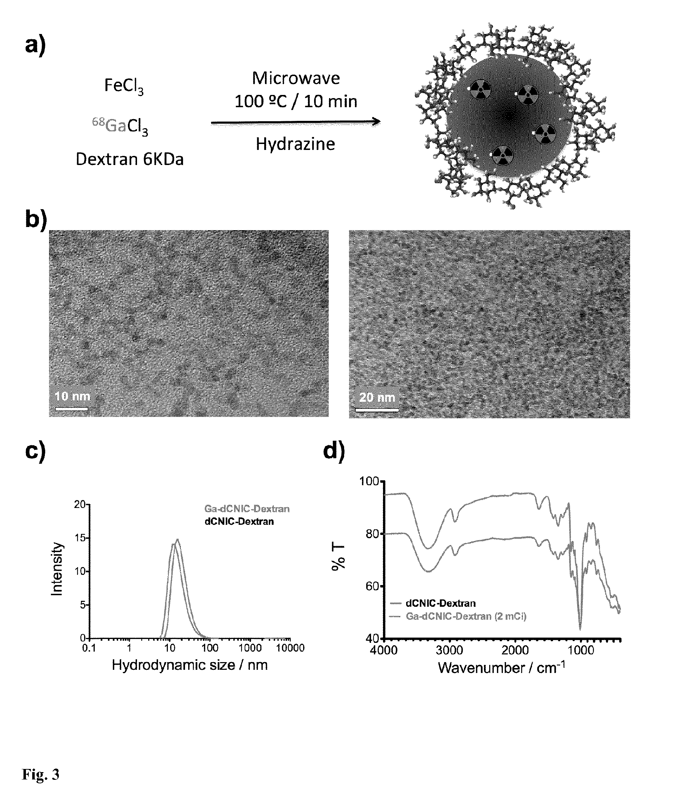

After demonstrating the feasibility of the approach for the synthesis of T₁ Fe₃O₄ nanoparticles, the authors applied the same synthetic approach for a further goal, the doping of the nanoparticle's core with a radioisotope, in particular a short half-life radioisotope such as ⁶⁸Ga, by using the MW technology that facilitates a reaction time frame window brief enough to prepare radiopharmaceuticals with short-lived isotopes. For that, the authors used the same reaction conditions already stated in Example 2 but with the addition of an activity of 2 mCi directly from the elution of a ⁶⁸Ge/⁶⁸Ga generator (Figure 3a). This elution was carried out with diluted HCl (0.05 M) (see example 3).

The results summarize in table 2 underlies two aspects. First, the reproducibility of the method is not affected by the different amounts of ⁶⁸Ga incorporated from the generator, and second, the method can be easily applied for higher activities extending the field of applications to large animal models or even to human studies.

After the complete decay of ⁶⁸Ga, the nanoparticles were fully characterized and compared to "cold" Dextran nanoparticles (particularly to check if any of the properties as T₁ contrast agents was affected due to the doping of the nanoparticles). As expected, TEM images show extremely small nanoparticles and well dispersed. Core size for ⁶⁸Ga-dCNIC-Dext was 2.2 ± 0.2 by TEM and 1.9 by XRD (Figure 3b and Figure 6). As expected, the hydrodynamic sizes were comparable to those of the cold particles (Table 1). Figure 3c shows a size parity between the two types of nanoparticles (N = 5) that clearly demonstrates that there is no appreciable structural change due to the incorporation of the radioisotope. The same similitude is obtained in the surface composition, as Figure 3d collects for the bands corresponding to dextran and Fe₃O₄.

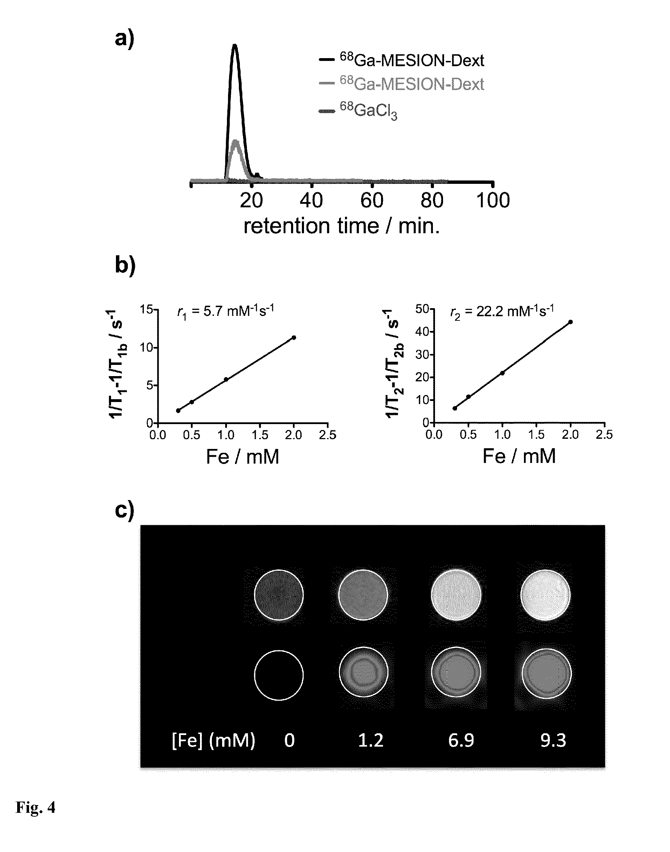

Additionally to the double purification steps, the authors carried out the typical quality control for nano-radiochemicals by gel filtration HPLC. Figure 4a shows the chromatogram for ⁶⁸Ga-dCNIC-Dextran in the UV channel at 380 nm and in the radioactivity channel, compared to what is obtained when free ⁶⁸GaCl₃ is injected into the column. This chromatogram reveals two aspects; one that the radioactivity peak is observed at the same retention time that the signal for nanoparticulate iron oxide, i.e. the radioactive signal is integrated in a nanoparticle; and, Secondly, that this elution pattern indicates that the radiation source does not derive from free radiotracer, which is not eluted quickly from these columns (figure 4a).

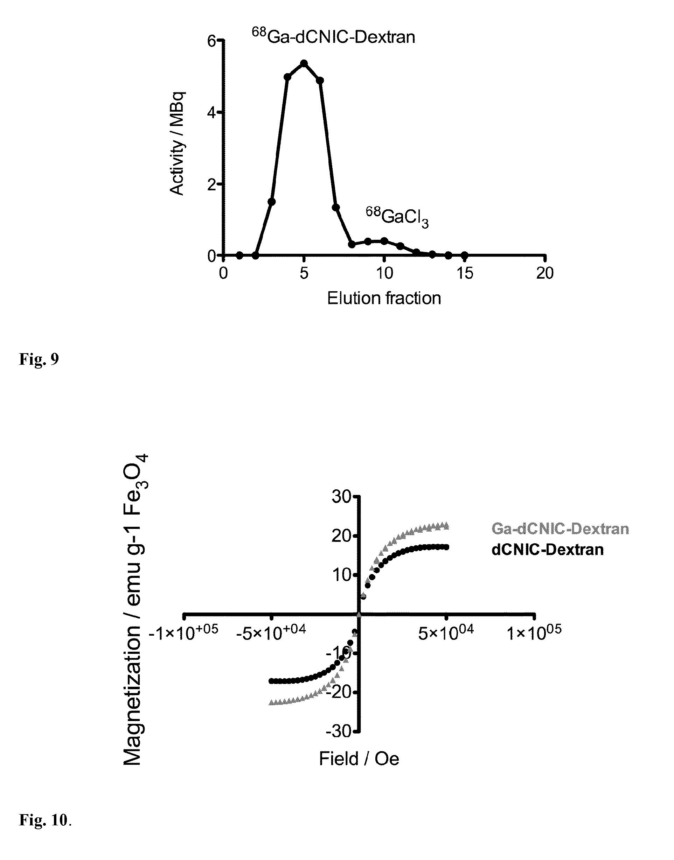

Next the authors determined whether or not the incorporation of ⁶⁸Ga affects the relaxation values they found for MEION. Figure 4b clearly answers that question, showing similar values for *r*₁ (5.7 mM⁻¹s⁻¹) and *r*₂ (22.2 mM⁻¹s⁻¹) and an *r*₂/*r*₁ ratio of 3.9, and thus, confirming that the magnetic properties of dCNIC are not affected. This was also checked by measuring the magnetic behavior of dCNIC with a SQUID. As it can be seen the results are almost identical to those for the "cold" nanoparticles (Figure 10).

With these results the authors continued by checking that the physicochemical characteristics of these nano-radiopharmaceuticals were suitable for high quality cardiovascular imaging. For that purpose, several phantoms with increasing amounts of ⁶⁸Ga-Dext nanoparticles were prepared. Both, PET images and T1-weighted MRI (acquired 24 h later) showed a correlation between signal intensity and the Fe in the preparation, which in turn is directly associated to the amount of doped Gallium in the nanoparticle (Figure 4c). As far as we know, this the first demonstration of "hot spot" PET-MRI imaging with a dual particle based on iron oxide.

Thus, a third aspect of the invention refers to a metal oxide nanoparticle, preferably an iron oxide nanoparticle, which comprises:
a. A surface layer of a biodegradable hydrophilic polymer; and
b. A core comprising a metal oxide, preferably a metal oxide selected from the following list of metals Fe, Al, B, Co, Zn, Ni, Zr, Ga, Y and Yb, more preferably the metal oxide is an iron oxide (such as FeO, Fe₃O₄, Fe₄O₅, Fe₂O₃ or FeO₃, still more preferably the iron oxide is Fe₃O₄), and radionuclide suitable for medical imaging;
wherein the nanoparticle is characterized by having the following values:
a. r1 is from 3-10 mM⁻¹s⁻¹as measured by relaxometry,
b. r2 is from 11-40 mM⁻¹s⁻¹as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 20 +/-10 as measured by a Superconducting Quantum Interference Device (SQUID).

A preferred embodiment of the third aspect of the invention refers to a metal oxide nanoparticle, preferably an iron oxide nanoparticle, which comprises:
a. A surface layer of a biodegradable hydrophilic polymer, preferably dextran (6kDal); and
b. A core comprising a metal oxide, preferably a metal oxide selected from the following list of metals Fe, Al, B, Co, Zn, Ni, Zr, Ga, Y and Yb, more preferably the metal oxide is an iron oxide (such as FeO, Fe₃O₄, Fe₄O₅, Fe₂O₃ or FeO₃, still more preferably the iron oxide is Fe₃O₄), and radionuclide selected from the list consisting of C-11, N-13, O-15, F-18, Cu-64, Ga-68, Ga-67, Tc-99, Ho-166, In-111, Lu-177, Yb-169, Bi-213, Er-169, Y-90 and T1-201 and Zr-89,
wherein the nanoparticle is characterized by having the following values:
a. r1 is from 3-10 mM⁻¹s⁻¹as measured by relaxometry
b. r2 is from 11-40 mM⁻¹s⁻¹as measured by relaxometry
c. a core size of from 1.5 - 3 nm, according to Transmission Electron Microscopy measurement,
d. a hydrodynamic size of from 20± 5 nm by DLS, and
e. a superparamagnetic behavior with a value of magnetic saturation (Ms) of from 22 +/-10 as measured by a Superconducting Quantum Interference Device (SQUID).

Another preferred embodiment of the third aspect of the invention refers to a metal oxide nanoparticle, preferably an iron oxide nanoparticle, which comprises:
a. A surface layer of a biodegradable hydrophilic polymer, preferably dextran (6 kDal); and
b. A core comprising a metal oxide, preferably a metal oxide selected from the following list of metals Fe, Al, B, Co, Zn, Ni, Zr, Ga, Y and Yb, more preferably the metal oxide is an iron oxide (such as FeO, Fe₃O₄, Fe₄O₅, Fe₂O₃ or FeO₃, still more preferably the iron oxide is Fe₃O₄), and radionuclide selected from the list consisting of C-11, N-13, O-15, F-18, Cu-64, Ga-68, Ga-67, Tc-99, Ho-166, In-111, Lu-177, Yb-169, Bi-213, Er-169, Y-90 and T1-201 and Zr-89, preferably Ga-68 or Zr-89.
wherein the nanoparticle is characterized by having the following values:
a. r1 is from 3-10 mM⁻¹s⁻¹as measured by relaxometry
b. r2 is from 11-40 mM⁻¹s⁻¹as measured by relaxometry
c. a core size of from 1.5 - 3 nm, according to Transmission Electron Microscopy measurement,
d. a hydrodynamic size of from 20± 5 nm by DLS, and
e. a superparamagnetic behavior with a value of magnetic saturation (Ms) of from 22 +/-2 as measured by a Superconducting Quantum Interference Device (SQUID).

Another preferred embodiment of the third aspect of the invention refers to a metal oxide, preferably an iron oxide nanoparticle, which comprises:
a. A surface layer of dextran; and
b. A core comprising an iron oxide (such as FeO, Fe₃O₄, Fe₄O₅, Fe₂O₃ or FeO₃, still more preferably the iron oxide is Fe₃O₄), and a radionuclide selected from the list consisting of Zr-89 and Ga-68, preferably Ga-68.
wherein the nanoparticle is characterized by having the following values:
a. r1 is from 3-10 mM⁻¹s⁻¹as measured by relaxometry
b. r2 is from 11-40 mM⁻¹s⁻¹as measured by relaxometry
c. a core size of from 1.5 - 3 nm, according to Transmission Electron Microscopy measurement,
d. a hydrodynamic size of from 20± 5 nm by DLS, and
e. a superparamagnetic behavior with a value of magnetic saturation (Ms) of from 22 +/-2 as measured by a Superconducting Quantum Interference Device (SQUID).

In addition and as already stated, the nanoparticles of the third aspect of the invention can be easily and reproducibly obtained by using the method detailed above wherein said method is not affected by the different amounts of ⁶⁸Ga incorporated.

Thus, a fourth aspect of the invention refers to a preparation method of metal oxide nanoparticles, preferably metal oxides from the following metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C, preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃,
   b. a biodegradable hydrophilic polymer, wherein preferably the hydrophilic polymer is dextran (preferably of 6kDal),
   c. a radionuclide suitable for medical imaging preferably selected from the group consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89, more preferably Ga-68 or Zr-89, and
   d. a strong base in a sufficient amount for the mixture to have a pH between 8 and 12, wherein preferably said strong base is Hydrazine hydrate.
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the fourth aspect of the invention refers to a preparation method of metal oxide nanoparticles, preferably metal oxides from the following metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), more preferably FeO3, comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol;
   c. a radionuclide suitable for medical imaging preferably selected from the group consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89, more preferably Ga-68 or Zr-89, in an amount between 0.5 mCi y 60 mCi, more preferably between 0.5 and 40 mCi; and
   d. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120°C with microwave irradiation at 220-300W for 3-11 min; and
c. Purifying the nanoparticles obtained in step b).

A preferred embodiment of the fourth aspect of the invention refers to a preparation method of metal oxide nanoparticles, preferably metal oxides from the following metals Fe, Al, B, Co, Zn, Ni, Zr, Ga, Y and Yb, more preferably iron oxide nanoparticles (such as FeO, Fe₃O₄, Fe₄O₅, Fe₂O₃ or FeO₃, still more preferably the iron oxide is Fe₃O₄), comprising the following steps:
a. Preparing a mixture in water or an alcohol having a boiling point above 90°C preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A metal salt, preferably an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. a biodegradable hydrophilic polymer, wherein preferably the hydrophilic polymer is dextran (preferably of 6kDal), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol.
   c. a radionuclide suitable for medical imaging preferably selected from the group consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89, more preferably Ga-68 or Zr-89, in an amount between 0.5 mCi y 60 mCi, more preferably between 0.5 and 40 mCi; and
   d. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature of about 100°C, with microwave irradiation at about 240W for about 10 min; and
c. Purifying the nanoparticles obtained in step b).

A fifth aspect of the invention refers to an iron oxide nanoparticle obtained or obtainable by the preparation method of the fourth aspect of the invention.

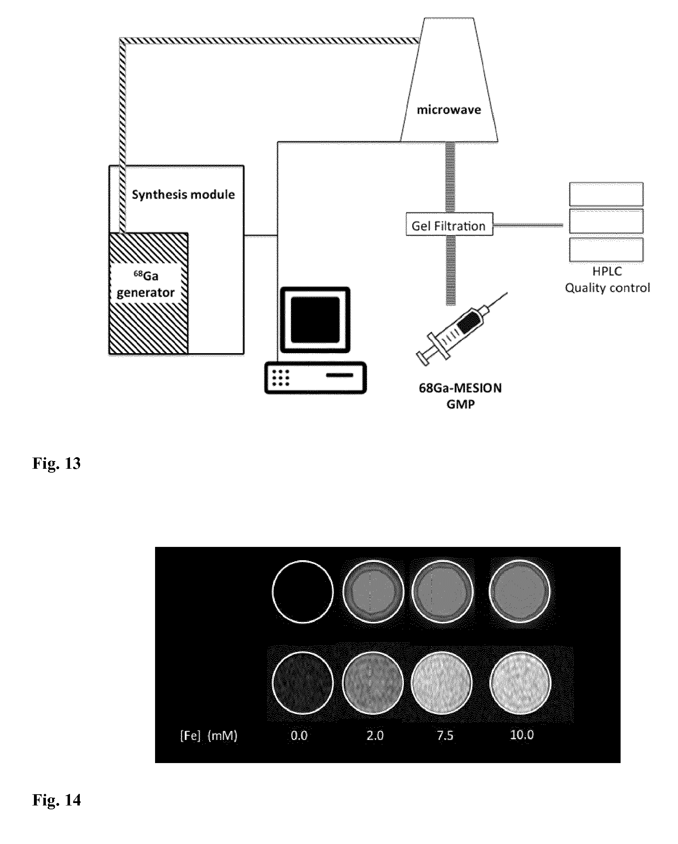

The new nanoparticles and the preparation method described herein is very encouraging for clinical translation. Nanometric size iron oxides have already been approved by the FDA and other regulatory agencies. The nanoparticles described herein are produced in a reproducible and controlled manner, and, even with their dual modality character included, the present proposal facilitates Good Manufacturing Practice (GMP) compliance in approved production facilities required for clinical use. Figure 13 illustrates the conceptual design of possible equipment suitable to carry out the preparation of GMP radiopharmaceutical.

Additionally, although this was not included in this work, we are not expecting safety or toxicological issues since the final formulations described herein include complete non-toxic components (iron oxide) and extremely low levels of radiolabelled compound (0.08 nmol of ⁶⁸Ga that after decay converts into biodegradable ⁶⁸Zn). Furthermore the thorough purification by size exclusion chromatography and ultrafiltration steps ensure that not free radioisotope and easy handling of pH and molarity of the final radiopharmaceutical-dCNIC formulation, as we saw in Figure 4a and Figure 9.

To get an insight on the expected long-term iron biodistribution of our dual nanoparticles the authors also synthesized ⁸⁹Zr-dCNIC-Dextran. The procedure and characterization steps for the preparation of this long half-life cyclotron-produced radioisotope were the same as described above. Figure 14 shows PET and MRI phantoms for the ⁸⁹Zr-dCNIC-Dextran with an 89% of radiolabeling efficiency and positive contrast. Size (20.1 nm), relaxivity values (*r*₂/*r*₁ of 3.8) and surface composition are similar to ⁶⁸Ga-dCNIC-Dextran (Table 1).

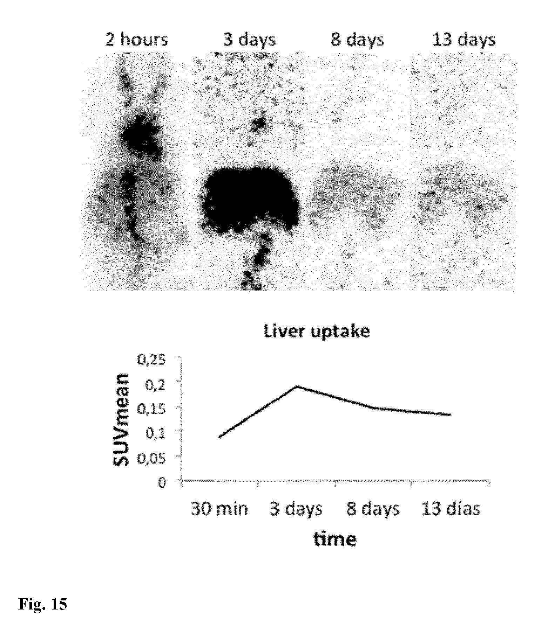

Due to the very small size, the small concentration of ⁶⁸Ga or ⁸⁹Zr, and the chelator-free nature of the approach, it was expected, and this was the case, the normal biodegradation route through the liver, as it was finally observed in all cases. Although Figure 15 summarizes the bio distribution of ⁸⁹Zr-based preparations in a period of 14 days after injection, one could expect the same biodistribution results for ⁶⁸Ga based nanoparticles or pure iron oxide coated nanoparticles of the same size and coating. Being the radionuclide integrated in the matrix of the nanoparticle, the size of the intravenously injected contrast agent and the nature of organic coating are both factors mainly determining the biodistribution and excretion pathways.

Thus a sixth aspect of the invention refers to the nanoparticles of the second, third or fifth aspect of the invention for use as a medicament, or for use in therapy or medical diagnosis.

A seventh aspect of the invention refers to the nanoparticles of the second, third or fifth aspect of the invention for use in a method of diagnosis and/or therapy capable of providing in vivo signals for PET (Positron Emission Tomography) and/or positive contrast MRI (Magnetic Resonance Imaging). An eight aspect of the invention refers to the use of the nanoparticles of the second, third or fifth aspect of the invention in a method capable of providing in vivo signals for PET (Positron Emission Tomography) and/or positive contrast MRI (Magnetic Resonance Imaging).

Finally in this work we describe an entirely new iron oxide-based radiopharmaceutical nanoplatform and present, after intravenous administration, extraordinary quality *in vivo* images by both MRI and PET. The present approach resulting in a completely new concept in the field, includes synthesis to prepare these radiolabelled superparamagnetic nanoparticles and, as novelty, comprises inserting the radionuclide directly in their cores. Our robust synthetic protocol overcomes many of the common limitations in probes for conventional PET imaging. With expedite chemistry and purification steps resulting in an optimized microwave-driven process, a new nuclear tracer mainly exampled with a short half-time radioisotope such as ⁶⁸Ga, is integrated in a biocompatible vascular-enhanced formulation. Both the methodology and the dual probe platform shown can be a breakthrough in the field, opening to many different libraries of compounds and applications addressing for instance quantification of different biological processes. The present solution can be easily adaptable to widespread clinical utilization, especially with the new generation of hybrid MRI/PET systems, to really take advantage of excellent spatial resolution, absolute quantification and superb sensitivity of each imaging modality.

A ninth aspect of the invention refers to the nanoparticles of the second, third or fifth aspect of the invention for use in a method of diagnosis and/or therapy via systemic administration, preferably intravenous administration, capable of providing in vivo signals for PET (Positron Emission Tomography) and/or positive contrast MRI (Magnetic Resonance Imaging).

The following examples have been inserted herein for illustration purposes only and thus do not limit the present invention.

### Examples

### Example 1. Materials and methods.

### 1.1. Synthesis of dCNIC-Dextran.

FeCl₃ x 6 H₂O (75 mg, 0.28 mmol) and Dextran (MW 6000 KDa, 140 mg, 0.02 mmol) were dissolved in distilled water (9 mL), inside the microwave-adapted flask, followed by an addition of 1 mL of hydrazine hydrate. The solution then was heated at 100°C, ramped in 54 s, for 10 minutes (240 W of power). After that, the mixture was cooled to 60 °C and purified. The reaction mixture was passed through a PD-10 column to eliminate excess of reagents and through ultrafiltration to remove the Dextran, not attached to the surface of the nanoparticles.

### 1.2. Synthesis of ⁶⁸Ga-dCNIC-Dextran.

FeCl₃ x 6 H₂O (75 mg, 0.28 mmol), Dextran (MW 6000 KDa, 140 mg, 0.02 mmol) and 74 MBq of ⁶⁸GaCl₃ in HCl (0.05 M) were dissolved in water (6 mL), inside the microwave-adapted flask, followed by an addition of 1 mL of hydrazine hydrate. The solution then was heated at 100°C in 54 s and the reaction processed for 10 minutes (240 W of power). After that the mixture was cooled to 60 °C and purified. The reaction mixture was passed through a PD-10 column for the purification from the unlabeled ⁶⁸Ga and hydrazine. After this purification 9 mL of ⁶⁸Ga-dCNIC-Dextran were obtained, with a total activity of 35.8 MBq, resulting in a radiolabeling yield of 93.4%.

### 1.3. Synthesis of ⁸⁹Zr-dCNIC-Dextran.

FeCl₃ x 6 H₂O (75 mg, 0.28 mmol), Dextran (MW 6000 KDa, 140 mg, 0.02 mmol) and 37 MBq of ⁶⁸Zr oxalate (in 0.1 M oxalic acid) were dissolved in water (6 mL), inside the microwave-adapted flask, followed by an addition of 1 mL of hydrazine hydrate. The solution then was heated at 100°C in 54 s and the reaction processed for 10 minutes (240 W of power). After that the mixture was cooled to 60 °C and purified. The reaction mixture was passed through a PD-10 column for the purification from the unlabeled ⁶⁸Zr and hydrazine. After this purification 9 mL of ⁶⁸ Zr-dCNIC- Dextran were obtained, with a radiolabeling yield of 89.0 %.

### 1.4. MRI relaxation properties of dCNIC samples.

Longitudinal and transversal relaxation times were measured for five concentrations of the nanoparticles in a Bruker Minispec mq60 at 1.5 T and 37 °C. Results were plotted as the R₁ and R₂ values versus the Fe concentration in mM.

### 1.5. MRI and PET imaging

Mice and rabbits were housed in specific (pathogen-free for mice) facilities at Centro Nacional de Investigaciones Cardiovasculares. Experimental procedures were approved by the Animal Care and Ethics Committee of the Centro Nacional de Investigaciones Cardiovasculares and regional authorities.

In vivo MRI in mice was performed on an Agilent/Varian scanner (Agilent, Santa Clara, USA) equipped with a DD2 console and active shielded 205/120 gradient insert coil with 130 mT/m maximum gradient strength and a combination of volume coil/two channel phased-array (Rapid Biomedical GmbH, Rimpar, Germany). Mice (20 g) were anesthetized with 2% Isoflurane and oxygen and positioned into a thermoregulated (38.7°C) mouse bed, with ophthalmic gel in their eyes to prevent the retinal drying.

Dynamic time-resolved MR angiography (MRA) was obtained with the following parameters TR MIN: 12.64 ms, TE MIN: 2.32 ms, FA 20, NA2, Matrix 192 X 128, MTC: FA 810 deg, duration 6 ms, offset 2000Hz.

In vivo MRI in rabbits was performed on an Achieva 3T Philips system with a 16 channels customized phased-array coil. Animal was placed inside the coil in supine position to reduce respiratory artefacts in the final acquired image. 3D spoiled gradient eco sequence (TR/TE/FA=7.5/3.77ms/10 degree) was performed with linear profile ordering and water selection fat suppression technique (PROSET 121). Using the effect that the contrast stay in the vascular tree for a long time, after reaching the steady-state of the contras distributed through the whole vasculature high resolution images were acquired with a pixel size of 0.5x0.5x0.8mm³, reconstructed to 0.4mm isotropic covering a volume of 120x120x200 mm³. Half-scan use used in RL and AP direction (0.85/0.7 respectively) with parallel acquisition factor of 1.9 with a resulting total acquisition time of 205s

*In vivo* PET/CT Imaging in mice was performed on a nanoPET/CT small animal imaging system (Mediso Medical Imaging Systems, Budapest, Hungary). List-mode PET data acquisition commenced immediately before starting a bolus of 2 mCi of ⁶⁸Ga-dCNIC-Dextran injection through the tail vein and continued for 2 hours. At the end of the PET, a microCT was performed for attenuation correction and anatomic reference. The dynamic PET images in a 105x105 matrix (frame rates: 3 x 10 min, 1 x 30 min, 1 x 60 min) were reconstructed using a Tera-Tomo 3D iterative algorithm. Acquisition and reconstruction were performed with proprietary Nucline software (Mediso, Budapest, Hungary). Qualitative Image analysis in mice and rabbits was performed using Osirix software (Pixmeo, Switzerland). The PET imaging of the phantom containing dilutions of ⁶⁸Ga-dCNIC-Dextran was acquired during 15 minutes to observe the increasing signal in correlation with the PET activity and magnetic resonance imaging.

In vivo PET/CT in rabbits data was acquired using a Philips Gemini TF 64 PET/CT scanner with time of flight capability. One rabbit (3.65 kg) was intravenously injected with a 45 MBq dosis of the ⁶⁸Ga-dCNIC-Dextran tracer and imaged one hour and one hour and a half after injection in a list-mode. PET images were reconstructed using a 3D-RAMLA algorithm with 3 iterations and 33 subsets and 2 mm isotropic voxel size. Two types of acquisition with a 20 cm FOV and 20 minutes accumulation (1 bed) and 100 cm (5 beds) and 1 minute/each with 50% overlapping. A reference CT scan used 140 kvp and 366 uA, helical scan with 1 mm pitch. Dynamic reconstruction was performed on the first imaging acquisition with 2 minutes frames. For this reconstruction, a voxel of 4 mm was used.

*In situ* PET phantom imaging containing dilutions of ⁶⁸Ga-dCNIC-Dextran was acquired during 15 minutes to observe the increasing signal in correlation with the PET activity and magnetic resonance imaging.

### 1.6. Field dependent magnetization.

Samples were prepared by adding 100 µL of each particles liquid suspension to a cotton piece. Then, the solvent was allowed to dry at room temperature overnight. The cotton, impregnated with the nanoparticles, was placed in a gelatine capsule for the magnetic characterization. Field dependent magnetization measurements were performed in a MPMS-5S SQUID Magnetometer (Quatum Design) at 250 K up to 5 T.

### 1.7. XRD

The crystal structure of the samples was identified by X-ray powder diffraction in a Bruker D8 Advance powder diffractometer using Cu Kα radiation with an energy-discriminator (Sol-X) detector. Patterns were registered within 10 and 80 in 2θ at 0.01 degrees per second.

The average crystallite size was calculated by Scherrer's equation using the half width of the (311) diffraction peak.

The XRD spectra correspond to an inverse spinel structure. The error in the crystallite sizes is 0.1 nm mainly due to the instrumental line width (Δ2θ = 0.11)

### Example 2. Microwave-assisted synthesis and physicochemical characterization of dCNIC-Dextran.

dCNIC-Dextran (in the context of the present invention CNIC is understood as "Consistent Nanoparticles with Imbedded radionuclide in a single Core") were synthesized by the combination of FeCl₃, Dextran (6 kDa) and Hydrazine hydrate in water. This mixture was subjected to very fast ramping (in 54 s) to 100 °C with MW irradiation at 240 W for 10 min. After purification by gel filtration chromatography, their physicochemical properties were studied. This approach rendered very small nanoparticles with a core size of about 2.1 ± 0.2 nm, according to TEM, and 2.6 nm of crystal size, according to XRD measurements (Figure 1a and Figure 6 respectively). Hydrodynamic size was also checked by DLS showing a value of 18.2 ± 2.5 nm (Figure 1b, Table 1). This is an extremely small size for an iron oxide nanoparticle coated by a large polymer as dextran. Surface composition of the dCNIC-Dextran was studied by FTIR showing the typical bands for dextran, particularly at 1000 cm⁻¹, and for iron oxide, at 400 cm⁻¹ (Figure 1c).

The authors of the present invention measured the magnetic properties of the synthesized particles using a Superconducting Quantum Interference Device (SQUID), the results are shown in Figure 1d, confirming the superparamagnetic behavior with a value of Ms of 17.2 emu/g Fe. These data confirm that due to the rapid heating and therefore nucleation in the MWS, extremely small Fe₃O₄ particles can be produced.

To examine the possibility of using the new synthesized dCNIC-Dextran particles as T₁ contrast agents in MRI, the relaxivity values, at 1.5 T and 37 °C, were measured. In order to use Fe₃O₄ nanoparticles as T₁ contrast agent, they must possess a high *r*₁ value and as low as possible *r*₂/*r*₁ ratio.

The experimental values and linear fitting are shown in figure 2a and Table 1 with a very high value for *r*₁ of 6.0 mM⁻¹s⁻¹ and a low *r*₂ value of 22.7 mM⁻¹s⁻¹. This gives a value for *r*₂ to *r*₁ ratio of 3.7 that should result in positive contrast. Of course there are two ways of getting small *r*₂/*r*₁ ratios, a very small *r*₂ or very high longitudinal relaxivity together with a modest *r*₂. This last option is the ideal one to produce good images, especially when the relaxivity values are as high as these, comparable or even higher than those exhibited by traditional Gd-based contrast agents. This high *r*₁ value of dCNIC-Dextran are clearly attributed, again, to the small size of the magnetic core leaving a large number of Fe³⁺ ions, with 5 unpaired electrons, on the surface of the dCNIC-Dextran.

To confirm the possibility of generating positive contrast with this new approach, several phantoms were prepared at different Fe concentrations and MRI recorded. As can be seen in figure 2b, with a T₁-weighted sequence, a strong positive contrast is obtained as the Fe concentration increase. As far as we know these are the first iron oxide nanoparticles synthesized by microwave radiation showing capabilities as T₁ contrast agent. Furthermore one of the strongest points of this technology is the reproducibility and rapidity to increase the production rate. As can be seen in figure 7 the DLS measurement for 5 different synthesis showed nanoparticles with the same hydrodynamic size and narrow size distribution (indicate time) and also with a very high amount of Dextran on the surface, ensuring colloidal stability (TGA data in Figure 8). The same is true for the main physicochemical properties of dCNIC-Dextran. Table 1 gathered some of the parameters for five repetitions of the synthesis confirming the reproducibility in all aspects, particularly in the relaxivity values and the *r*₂ to *r*₁ ratio, compulsory for their *in vivo* use.

**Table 1. Main physicochemical properties of dCNIC-Dextran and ⁶⁸Ga-dCNIC-Dextran.**

| | **HD size** | **TEM size** | **XRD** | **Ms** | ***r*₂** | ***r*₁** | **r2/r1** | **Activity** | **% Radiolab.** |
|---|---|---|---|---|---|---|---|---|---|
| **SAMPLE (N=5)** | (nm) | (nm) | (nm) | emu⁻¹g⁻¹ | (mM⁻¹s⁻¹) | (mM⁻¹s⁻¹) | | (mCi) | |
| **dCNIC-Dextran** | 18.2 ± 2.5 | 2.1 ± 0.2 | 2.6 | 17.2 | 22.7 ± 3.1 | 6.0 ± 0.2 | 3.7 | n.a. | n.a. |
| **⁶⁸Ga-dCNIC-Dextran** | 20.6 ± 2.4 | 2.2 ± 0.2 | 1.9 | 22.4 | 22.2 | 5.7 ± 0.1 | 3.9 | 1.03 | 93 |

### Example 3. Microwave-assisted synthesis and physicochemical characterization of ⁶⁸Ga-dCNIC-Dextran.

After demonstrating the feasibility of our approach for the synthesis of T₁ Fe₃O₄ nanoparticles, we applied it for a further goal, the doping of dCNIC core with a radioisotope, in particular a short half-life radioisotope such as ⁶⁸Ga, by using the MW technology that facilitates a reaction time frame window brief enough to prepare radiopharmaceuticals with short-lived isotopes. For that, we used the same reaction conditions already stated in Example 1 but with the addition of an activity of 2 mCi directly from the elution of a ⁶⁸Ge/⁶⁸Ga generator (Figure 3a). This elution was carried out with diluted HCl (0.05 M). After the reaction, the nanoparticles were purified twice. Initially, by gel filtration to eliminate the excess of free ⁶⁸Ga, that was not incorporated into the nanoparticles. The typical profile for such purification step is shown in figure 9, where the main peak due to the radioactivity of the nanoparticles can be clearly seen, followed by a smaller peak due to free ⁶⁸Ga in the eluent. In this purification step, all unreacted small molecules were also eliminated, such excess of FeCl₃ or hydrazine. The excess of dextran is co-eluted with the nanoparticles due to its large size. As a consequence, we used a second purification step through ultrafiltration with a 100 KDa cutoff. Since these two purification steps endures typically no longer than 5 minutes, no significant activity is lost during the process, besides free ⁶⁸Ga is completely eliminated ensuring lack of toxicity and enhanced imaging resolution.

The reproducibility of the method is also found in the activity of the synthesized ⁶⁸Ga-dCNIC, after the filtration and purification steps resulted in a radiolabeling mean (N=5) yield of 93.4 %. The present procedure thus reproducibly provides 9 mL of ⁶⁸Ga-dCNIC-Dextran particles with 1.03 mCi and 1.3 mg Fe/mL, a distinct scenario allowing *in vivo* PET and MRI with the same nanoparticles, as we shall demonstrate in the following sections. This consistent protocol was repeated with different ⁶⁸Ga activities, namely for 1 mCi, 1.7 mCi, 3 mCi and 3.6 mCi. The main physicochemical properties for each preparation are shown in table 2.

**Table 2. Main physicochemical parameters for 68Ga-dCNIC-Dextran samples with different activities.**

| **Sample** | **Size (nm)** | **TEM (nm)** | ***r*₂/*r*₁** | **Radiolabeling yield** |
|---|---|---|---|---|
| **68Ga-dCNIC-Dextran (1.0 mCi)** | 20,42 | 2 | 3.9 | 85,7 |
| **68Ga-dCNIC-Dextran (1.7 mCi)** | 24,66 | 1.9 | 3.8 | 82,8 |
| **68Ga-dCNIC-Dextran (3.0 mCi)** | 20,44 | 2.2 | 3.9 | 93,4 |
| **68Ga-dCNIC-Dextran (3.6 mCi)** | 22,89 | 2.3 | 3.9 | 90,4 |

The results summarize in the table underlies two aspects. First, the reproducibility of the method is not affected by the different amounts of ⁶⁸Ga incorporated from the generator, and second, the method can be easily applied for higher activities extending the field of applications to large animal models or even to human studies.

After the complete decay of ⁶⁸Ga, the nanoparticles were fully characterized and compared to "cold" dCNIC-Dextran (particularly to check if any of the properties as T₁ contrast agents was affected due to the doping of the nanoparticles). As expected, TEM images show extremely small nanoparticles and well dispersed. Core size for ⁶⁸Ga-dCNIC-Dext was 2.2 ± 0.2 by TEM and 1.9 by XRD (Figure 3b and Figure 6). As expected, the hydrodynamic sizes were comparable to those of the cold particles (Table 1). Figure 3c shows a size parity between the two types of nanoparticles (N = 5) that clearly demonstrates that there is no appreciable structural change due to the incorporation of the radioisotope. The same similitude is obtained in the surface composition, as Figure 3d collects for the bands corresponding to dextran and Fe₃O₄.

Additionally to the double purification steps, we carried out the typical quality control for nano-radiochemicals by gel filtration HPLC. Figure 4a shows the chromatogram for ⁶⁸Ga-dCNIC-Dextran in the UV channel at 380 nm and in the radioactivity channel, compared to what is obtained when free ⁶⁸GaCl₃ is injected into the column. This chromatogram reveals two aspects; one that the radioactivity peak is observed at the same retention time that the signal for nanoparticulate iron oxide, i.e. the radioactive signal is integrated in a nanoparticle; and, secondly, that this elution pattern indicates that the radiation source does not derive from free radiotracer, which is not eluted quickly from these columns (figure 4a).

Next the authors determined whether or not the incorporation of ⁶⁸Ga affects the relaxation values they found for MEION. Figure 4b clearly answers that question, showing similar values for *r*₁ (5.7 mM⁻¹s⁻¹) and *r*₂ (22.2 mM⁻¹s⁻¹) and an *r*₂/*r*₁ ratio of 3.9, and thus, confirming that the magnetic properties of dCNIC are not affected. This was also checked by measuring the magnetic behavior of dCNIC with a SQUID. As it can be seen the results are almost identical to those for the "cold" nanoparticles (Figure 10).

With these results the authors continued by checking that the physicochemical characteristics of these nano-radiopharmaceuticals were suitable for high quality cardiovascular imaging. For that purpose, several phantoms with increasing amounts of ⁶⁸Ga-dCNIC-Dext particles were prepared. Both, PET images and T₁-weighted MRI (acquired 24 h later) showed a correlation between signal intensity and the Fe in the preparation, which in turn is directly associated to the amount of doped Gallium in the nanoparticle (Figure 4c). As far as we know, this the first demonstration of "hot spot" PET-MRI imaging with a dual particle based on iron oxide.

### Example 4. In vivo imaging

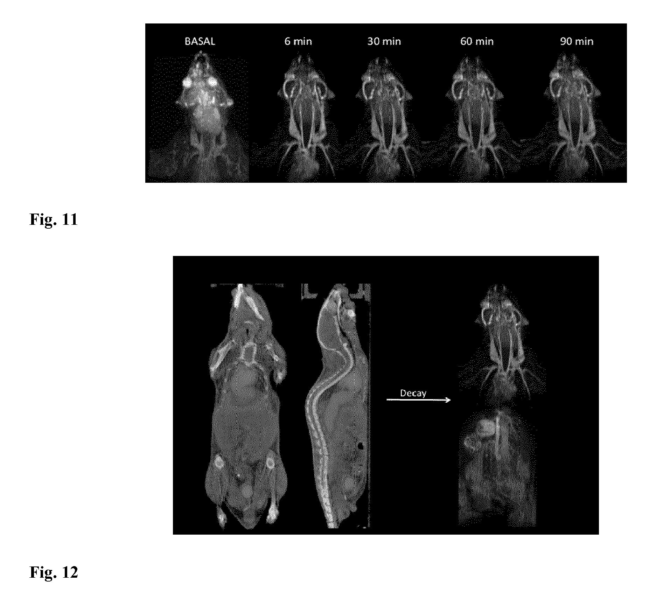

To demonstrate the use of ⁶⁸Ga-dCNIC-Dextran *in vivo* for positive contrast MRI, the authors performed several *in vivo* studies in mice and rabbits. For that, nanoparticles were injected at low dose, 2.2 mg Fe/ Kg, in healthy animals. After this injection, shorten T₁ relaxation times lead to higher signal intensity and excellent anatomical detail in MR angiographic (MRA) acquisitions. The views generated after fat suppression acquisitions and maximum intensity projection reconstructions clearly depicted some main vascular segments and small vessels (Figure 5 and Figure 11). The optimal angiographic sequence used for MRI acquisition in rabbits allows a remarkable high quality for iron oxide based MRA, demonstrated for small vessels 1 hour after the injection of the nanoparticles. This highlights another important aspect of these nanoparticles, due to their small sizes and coating nature they remain in circulation longer than most nanoparticles and much more than the typical radiopharmaceutical compounds. Figure 5 clearly shows an excellent signal enhancement at 6 minutes and 1 hour post-injection of the probe. The same result was found in mice, 90 minutes after the injection, MRA signal was not significantly different during this period (Figure 11). Thus, these nanoparticles are suitable for contrast-enhanced MRA providing an excellent signal (contrast) separation between vessels and perivascular tissues with reduced acquisition times and without the presence of susceptibility artifacts. Using NPs as contrast agent for MRA has the advantage to deal with the high concentration of contrast agent needed throughout image acquisition, and efficiently matched the conditions between scan time and blood residence time required for an optimal result. Using this long circulating T₁ iron oxide based contrast agents, MRA is simplified, data accumulation is possible and high quality of e.g. high quality imaging of coronaries is feasible.

The dual character of ⁶⁸Ga-dCNIC-Dextran opens new possibilities for dual PET/MRI systems, namely opening the possibility of simultaneous (or consecutive) quantification of different pathologic processes as tumor angiogenesis in vivo. Figure 6 shows PET and MRI images for a rabbit after the injection of ⁶⁸Ga-dCNIC-Dextrane (1.2 mCi). In contrast to the use of traditional chelators, ⁶⁸Ga-dCNIC-Dextran remained in circulation for a time sufficient for the detailed imaging of vessels in PET.

⁶⁸Ga-dCNIC-Dextran can be detected in existing hybrid dual modality instruments enabling the possibility for an exceptional range of cross-validation experiments through which to gain new quantitative and qualitative insights into pathophysiological mechanisms of diseases such as angiogenesis, inflammation, thrombosis, myocardial infarction, renal failure, atherosclerotic plaque, etc. Figure 5b confirms the in vitro results, after the decay of the radionuclide the NPs can be used for positive contrast MRI with the same results previously shown for the cold nanoparticles. The same situation was found in mice as Figure 12 demonstrates.

## Claims

1. An iron oxide nanoparticle which comprises:
a. A surface layer of a biodegradable hydrophilic polymer; and
b. A core comprising an iron oxide and radionuclide suitable for medical imaging;
wherein the nanoparticle is **characterized by** having the following values:
a. r1 is from 3-10 mM⁻¹s⁻¹ as measured by relaxometry,
b. r2 is from 11-40 mM⁻¹s⁻¹ as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 20 +/-10 as measured by a Superconducting Quantum Interference Device (SQUID).

2. The iron oxide nanoparticle of claim 1 wherein the radionuclide is selected from the list consisting of C-11, N-13, O-15, F-18, Cu-64, Ga-68, Ga-67, Tc-99, Ho-166, In-111, Lu-177, Yb-169, Bi-213, Er-169, Y-90 and T1-201 and Zr-89.

3. The iron oxide nanoparticle of any of claims 1 or 2 wherein the radionuclide is Ga-68 or Zr-89.

4. The iron oxide nanoparticle of any of claims 1-3, wherein the iron oxide is selected from the group consisting of FeO, Fe₃O₄, Fe₄O₅, Fe₂O₃ or FeO₃.

5. The iron oxide nanoparticle of any of claims 1-4 wherein the biodegradable hydrophilic polymer is dextran.

6. An iron oxide nanoparticle which comprises:
a. A surface layer of dextran; and
b. A core comprising an iron oxide selected from the group consisting of FeO, Fe₃O₄, Fe4O5, F2O3 or FeO3, and radionuclide selected from the group consisting of Ga-68 or Zr-89;
wherein the nanoparticle is **characterized by** having the following values:
a. r1 is from 3-10 mM⁻¹s⁻¹as measured by relaxometry,
b. r2 is from 11-40 mM⁻¹s⁻¹as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 22 +/- 2 as measured by a Superconducting Quantum Interference Device (SQUID).

7. A preparation method of iron oxide nanoparticles comprising the following steps:
a. Preparing a mixture in water or an alcohol selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
a. an iron salt,
b. a radionuclide suitable for medical imaging,
c. a biodegradable hydrophilic polymer, and
d. a strong base in a sufficient amount for the mixture to have a pH between 8 and 12,
b. Subjecting said mixture to fast ramping in less than three minutes to a temperature between 90 and 120°C with microwave irradiation at 220-300W for 3-11 min; and
c. Purifying the nanoparticles obtained in step b).

8. The preparation method of iron oxide nanoparticles of claim 6 wherein the radionuclide is selected from the list consisting of C-11, N-13, O-15,F-18, Cu-64, Ga-68, Ga-67, Tc-99, Ho-166, In-111, Lu-177, Yb-169, Bi-213, Er-169, Y-90 and T1-201 and Zr-89.

9. The preparation method of iron oxide nanoparticles of any of claims 7 or 8, wherein the iron salt is selected from the group consisting of FeCl₃, Fe(SO4), Fe₂(SO4)₃, Fe(NO₃)₃, FeCO₅, Fe(OH)₂ and Fe(OH)₃.

10. The preparation method of iron oxide nanoparticles of any of claims 7 -9, wherein the radionuclide is Ga-68 or Zr-89.

11. The preparation method of iron oxide nanoparticles of any of claims 7-10, wherein the mixture of step a) is subjected in step b) to fast ramping in less than one minute to about 100 °C with microwave irradiation at 220-240W for 8-11 min.

12. The preparation method of iron oxide nanoparticles of any of claims 7-10, wherein the mixture of step a) is subjected in step b) to fast ramping in less than one minute to about 100 °C with microwave irradiation at about 240W for about 10 min.

13. A nanoparticle as defined in any of claims 1-6 for use in therapy or medical diagnosis.

14. A nanoparticle as defined in any of claims 1-6 for use in a method of diagnosis and/or therapy capable of providing in vivo signals for PET (Positron Emission Tomography) and/or positive contrast MRI (Magnetic Resonance Imaging).

15. A nanoparticle as defined in any of claims 1-5 for use in a method of diagnosis and/or therapy via systemic administration, preferably intravenous administration, capable of providing in vivo signals for PET (Positron Emission Tomography) and/or positive contrast MRI (Magnetic Resonance Imaging).
